(19) Europäisches Patentamt
European Patent Office
Office européen des brevets

(11) EP 2 062 575 B1

# (12) EUROPEAN PATENT SPECIFICATION

(45) Date of publication and mention of the grant of the patent:
**15.02.2012 Bulletin 2012/07**

(21) Application number: **07807094.3**

(22) Date of filing: **11.09.2007**

(51) Int Cl.:
***A61K 31/137*** *(2006.01)* ***A61K 9/70*** *(2006.01)*
***A61K 47/04*** *(2006.01)* ***A61K 47/14*** *(2006.01)*
***A61K 47/32*** *(2006.01)* ***A61K 47/38*** *(2006.01)*
***A61P 9/02*** *(2006.01)*

(86) International application number:
**PCT/JP2007/067685**

(87) International publication number:
**WO 2008/032718 (20.03.2008 Gazette 2008/12)**

## (54) ADHESIVE PREPARATION COMPRISING DESGLYMIDODRINE

KLEBENDE ZUBEREITUNG MIT DESGLYMIDODRIN

PREPARATION ADHESIVE COMPRENANT DE LA DESGLYMIDODRINE

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **11.09.2006 JP 2006246188**
**20.06.2007 JP 2007163208**

(43) Date of publication of application:
**27.05.2009 Bulletin 2009/22**

(73) Proprietor: **Sekisui Chemical Co., Ltd.**
**Osaka 530-8565 (JP)**

(72) Inventors:
- **UDAGAWA, Hiroko**
  **Amagasaki-shi,**
  **Hyogo, 661-8564 (JP)**
- **KOMODA, Toshikazu**
  **Amagasaki-shi,**
  **Hyogo, 661-8564 (JP)**
- **HAMABE, Masaru**
  **Amagasaki-shi,**
  **Hyogo, 661-8564 (JP)**
- **NODA, Yukihiko**
  **Amagasaki-shi,**
  **Hyogo, 661-8564 (JP)**
- **SHIBATA, Sakiko**
  **Chuo-ku,**
  **Tokyo, 103-0027 (JP)**
- **YAMADA, Masashi**
  **Chiyoda-ku,**
  **Tokyo, 101-0047 (JP)**
- **KAWASHIMA, Akihiro**
  **Odawara-shi,**
  **Kanagawa, 250-0862 (JP)**
- **SUZUKI, Hiroto**
  **Chiyoda-ku,**
  **Tokyo, 101-0047 (JP)**

(74) Representative: **Merkle, Gebhard**
**TER MEER STEINMEISTER & PARTNER GbR,**
**Patentanwälte**
**Mauerkircherstrasse 45**
**81679 München (DE)**

(56) References cited:
**EP-A1- 1 498 118** **EP-A2- 0 450 986**
**JP-A- 11 139 968** **JP-A- 2003 300 873**
**US-A1- 2002 147 232**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

Technical Field

The present invention relates to transdermal patches for transdermally administering 2-amino-1-(2', 5'-dimethoxyphenyl) ethanol (hereinafter referred to as "DMAE" also known as desglymidodrine) or its pharmacologically acceptable salt.

Background Art

It has been known that the DMAE increases blood pressure by selectively stimulating an $\alpha_1$-receptor and constricting a peripheral blood vessel, and midodrine, which is a prodrug of the DMAE activated by glycine, is used for treatment of essential hypotension and orthostatic hypotension. Further, in a recent clinical practice, since attention has been paid to a smooth muscle contractile function resulting from the stimulation of the $\alpha_1$-receptor by the DMAE, the DMAE or the midodrine is expected to be applicable for treatment of abdominal pressure-induced incontinence.
Currently in Japan, an oral agent composed of midodrine hydrochloride as a major component, being hydrochloride of the midodrine, is commercially available as a medicine or drug composed of the midodrine mentioned above; with regard to its general dosage and administration, a 2-mg tablet is to be dosed twice in a day. However, there has arisen a problematic risk that, after a dosage of the oral agent mentioned above, a blood concentration of the DMAE, being midodrine hydrochloride metabolite, is rapidly increased, resulting in occurrence of supine hypertension as a side effect.
In order to remedy such a drawback as described above, there has been proposed a method in which the DMAE is transdermally administered by applying a transdermal patch to a human skin, the patch having a plaster layer containing the DMAE and an adhesive. Administration of the DMAE by means of such transdermal patch includes advantages that: (1) a rapid increase in blood concentration is less likely to occur because the DMAE can be absorbed through the skin at a slow speed over an elongated period of time; (2) a primary metabolism of the DMAE within a hepar is avoided and improves bioavailability; and (3) even when a side effect occurs, the administration of the DMAE can be immediately stopped by removing the transdermal patch away from the skin.
With regard to the transdermal patch mentioned above, Patent Document 1 proposes a transdermal therapeutic drug containing DMAE or its salt, and also discloses a transdermal therapeutic drug that contains, as additives for increasing a transdermal absorbency of the DMAE or its salt, a wetting agent or moisturizing agent such as glycerin, propylene glycol, N-methyl-2-pyrrolidone, isopropyl myristate, capric acid and myristic acid, and a pH adjuster made of a basic substance such as monoethanolamine, diethanolamine and triethanolamine.
In the transdermal therapeutic drug described above, however, there has been a problem that preservation stability of the DMAE or its salt becomes insufficient depending on an adhesive to be used, a content of the DMAE or its salt decreases, or stimulation on the skin is caused by a resultant degradation product of the DMAE or its salt while the patch is applied on the skin.
Further, Patent Document 2 proposes a transdermal patch in which a noncrosslinked adhesive layer (A) is laminated on one surface of a support, the adhesive layer (A) containing DMAE or its pharmacologically acceptable salt, and a crosslinked adhesive layer (B) is laminated on the noncrosslinked adhesive layer (A).
In the case of the transderinal patch described in Patent Document 2, there arose a problem that, during storage of the patch, when the DMAE or its pharmacologically acceptable salt contained in the noncrosslinked adhesive layer (A) is diffused into the crosslinked adhesive layer (B) or when an crosslinking agent, not reacted yet, which is contained in the crosslinked adhesive layer (B) is diffused into the noncrosslinked adhesive layer (A), a content of the DMAE or its pharmacologically acceptable salt in the transdermal patch is reduced because the DMAE or its pharmacologically acceptable salt reacts with the crosslinking agent being not reacted yet. Further, this particular transdermal patch suffered the disadvantage that, because of an intricate production process of laminating the adhesive layers in two layers, homogeneity in quality was hard to secure and also a cost of manufacture was expensive.
Patent Document 1 Japanese Patent No. 3571511
Patent Document 2 Japanese Patent Application Laid-Open Publication No. 2003-300873 - Family member of the Japanese Application is EP 14 98 118 A.

Disclosure of Invention

Problems to Be Solved by the Invention

It is an object of the present invention to provide a transdermal patch which is superior in preservation stability and transdermal absorbency of DMAE or its pharmacologically acceptable salt and also assures ease of manufacture.

Means for Solving the Problems

The transdermal patch of the present invention is the one in which a plaster layer is integrally laminated on one surface of a support, the plaster layer including: 2-amino-1-(2', 5'-dimethoxyphenyl) ethanol or its pharmacologically acceptable salt; 40 to 96% by weight of acrylic adhesive prepared by copolymerizing monomers respectively containing 30 to 99% by weight of alkyl methacrylate having an alkyl group with a carbon number of 6 to 22 and 1 to 70% by weight of alkyl acrylate having an alkyl group with a carbon number of 2 to 20; and 3 to 40% by weight of fatty acid ester prepared by dehydro-condensing saturated fatty acid having an alkyl group with a carbon number of 10 to 20 and saturated aliphatic monohydric alcohol having an alkyl group with a carbon number of 2 to 20, wherein solubility of the 2-amino-1-(2', 5'-dimethoxyphenyl) ethanol or its pharmacologically acceptable salt is 0.05 to 5 g at 25°C with respect to the fatty acid ester.

Contained in the plaster layer of the transdermal patch described above is 2-amino-1-(2', 5'-dimethoxyphenyl) ethanol (DMAE) or its pharmacologically acceptable salt. Typical examples of the pharmacologically acceptable salt of the DMAE described above include the DMAE and the salt resulting from reaction with inorganic acid or organic acid. Typical examples of such inorganic acids include hydrochloric acid, hydrobromic acid, nitric acid, sulfuric acid and phosphoric acid, while typical examples of the organic acids include formic acid, acetic acid, trifluoro acid, propionic acid, lactic acid, tartaric acid, oxalic acid, fumaric acid, maleic acid, citric acid, malonic acid and methanesulfonic acid.

The content of the DMAE or its pharmacologically acceptable salt (hereinafter collectively referred to as "DMAEs") in the plaster layer described above is preferably 0.5 to 40% by weight, more preferably 2 to 30% by weight, even more preferably 5 to 30% by weight, and most preferably 5 to 25% by weight. This is because, when the content of the DMAEs is small in the plaster layer, the amount of transdermal absorption of the DMAEs becomes small, and so when an area of the transdermal patch is not enlarged, the blood concentration of the DMAE may sometimes not increase to a desired range; and also because, when the content of the DMAEs is large in the plaster layer, a crystal of the DMAEs is liable to be excessively separated out to the plaster layer, and resultantly the patch may encounter a decrease in adhesion or in diffusibility of the DMAEs.

The transdermal patch of the present invention is required to increase the blood concentration of DMAE to a desired range for use as a therapeutic drug; in order to obtain such effect, it is required that a sufficient amount of DMAEs be stably contained in the plaster layer and that the DMAEs in a dissolved state be diffused into the plaster layer, so that the DMAEs are continuously delivered to a surface applied to the skin. In order to construct such a plaster layer being superior in preservation stability and transdermal absorbency of the DMAEs, the transdermal patch of the present invention is so arranged that the adhesive constituting the plaster layer uses an acrylic adhesive prepared by copolymerizing a respectively prescribed amount of alkyl methacrylate and alkyl acrylate; the alkyl methacrylate in a state of a monomer is superior in preservation stability of the DMAEs but is inferior in solvency and diffusibility of the DMAEs, while the alkyl acrylate in a state of a monomer is inferior in preservation stability of the DMAEs but is superior in solvency and diffusibility of the DMAEs. When such adhesive is used, the insufficient solvency and diffusibility of the DMAEs with the alkyl methacrylate being used alone are complemented by the superior solvency and diffusibility which is the advantage carried by the alkyl acrylate, while the problem of preservation stability of the DMAEs which is the disadvantage suffered by the alkyl acrylate is solved by the superior preservation stability of the DMAEs which is the advantage carried by the alkyl methacrylate.

In the case of the acrylic adhesive described above, therefore, the preservation stability of the DMAEs is very good, while a sufficient amount of the DMAEs can be dissolved to exhibit a drug effect, and also compatibility is excellent with respect to the fatty acid ester which is contained to improve the transdermal absorbency of the DMAEs. In addition, the alkyl methacrylate component and the alkyl acrylate component which constitute the acrylic adhesive differ in their respective contribution to an adhesion property, so that when a composition of these components is adjusted, the adhesive strength possessed by the plaster layer can be easily adjusted.

The alkyl methacrylate described above is limited to what has an alkyl group with a carbon number of 6 to 22. In the case of the alkyl methacrylate with an alkyl group having a carbon number of 5 or less than 5, a plasticizing effect by the alkyl group decreases to result in the acrylic adhesive becoming harder, which invites shortage of elasticity as an adhesive. When the alkyl methacrylate has an alkyl group with a carbon number of 23 or more than 23, an intertanglement becomes stronger between alkyl groups in the alkyl methacrylate component contained in the acrylic adhesive, leading to excessively strong viscosity of the adhesive. Therefore, when the carbon number of the alkyl group in the alkyl methacrylate is either larger or smaller, the adhesion of the acrylic adhesive to the skin decreases. Further, when the carbon number of the alkyl group in the alkyl methacrylate is too large, the solvency and diffusibility of the DMAEs may sometimes decrease, so that the carbon number of the alkyl group is preferably 6 to 16.

The above-described alkyl methacrylate having the alkyl group with a carbon number of 6 to 22 is not specifically limited, but typical examples include hexyl methacrylate, octyl methacrylate, decyl methacrylate, dodecyl methacrylate, tridecyl methacrylate, octadecyl methacrylate, 2-ethylhexyl methacrylate and tetradecyl methacrylate, and in particular, the 2-ethylhexyl methacrylate and the dodecyl methacrylate are preferred. The alkyl methacrylate may be used either alone or in combination of two or more kinds.

When the content of alkyl methacrylate in the monomer serving as a raw material for an acrylic adhesive is small, reactivity of the acrylic adhesive to the DMAEs becomes higher, resulting in lower preservation stability of the DMAEs and lower elasticity of the acrylic adhesive, and thus an adhesive transfer to the skin is liable to occur. When the content of alkyl methacrylate in the monomer serving as a raw material for an acrylic adhesive is large, the solvency and diffusibility of the DMAEs become lower in the plaster layer, or the plaster layer becomes hard, resulting in decrease in an initial adhesion to the skin. Therefore, the content of alkyl methacrylate in the monomer serving as a raw material for an acrylic adhesive is limited to 30 to 99% by weight, preferably 50 to 99% by weight, and more preferably 50 to 95% by weight.

The alkyl acrylate described above is limited to what has an alkyl group with a carbon number of 2 to 20. This is because, either when the alkyl group of the alkyl acrylate is a methyl group or when a carbon number is 21 or more than 21, the elasticity or the viscosity of the acrylic adhesive decreases, and so in either case the adhesive strength to the skin decreases.

The above-described alkyl acrylate with an alkyl group having a carbon number of 2 to 20 is not specifically limited, but typical examples include ethyl acrylate, propyl acrylate, butyl acrylate, isobutyl acrylate, hexyl acrylate, octyl acrylate, decyl acrylate, dodecyl acrylate, tridecyl acrylate, octadecyl acrylate and 2-ethylhexyl acrylate; and in particular, the 2-ethylhexyl acrylate is preferred. The alkyl acrylate may be used either alone or in combination of two or more kinds.

When the content of alkyl acrylate in the monomer serving as a raw material for an acrylic adhesive is small, the solvency and diffusibility of the DMAEs become lower in the plaster layer, or the plaster layer suffers lowered initial adhesion to the skin. When the content of alkyl acrylate is large, the preservation stability of the DMAEs contained in the plaster layer decreases, or internal cohesion of the plaster layer decreases; so when the plaster layer is excessively plasticized by fatty acid ester as described below, an adhesive transfer to the skin occurs when the transdermal patch is removed away from the skin. Therefore, the content of the alkyl acrylate is limited to 1 to 70% by weight, and preferably 1 to 50% by weight.

Preferably, the acrylic adhesive described above is one that is prepared by copolymerizing the monomers respectively containing 70 to 95% by weight of 2-ethylhexyl methacrylate and 1 to 30% by weight of 2-ethylhexyl acrylate, and more preferable is the acrylic adhesive being prepared by copolymerizing the monomers respectively containing 70 to 95% by weight of 2-ethylhexyl methacrylate, 1 to 20% by weight of the 2-ethylhexyl acrylate and 4 to 29% by weight of the dodecyl methacrylate. This is particularly because, when compounded with the fatty acid ester as described below, the acrylic adhesive exhibits more excellent preservation stability and transdermal absorbency of the DMAEs as well as excellent plastering ability to the skin.

When the content of the acrylic adhesive is small in the plaster layer described above, the adhesive strength to the skin decreases; when the content is large, sufficient amounts of DMAEs and fatty acid ester cannot be compounded in order to obtain a desired blood concentration of the DMAE, so that the content is limited to 40 to 96% by weight, preferably 45 to 90% by weight, and more preferably 55 to 85% by weight.

As far as the preservation stability and the transdermal absorbency of the DMAEs and the plastering ability to the skin are not spoiled, the acrylic adhesive may be prepared by copolymerizing the monomers respectively containing a monomer other than alkyl methacryl with an alkyl group having a carbon number of 6 to 22 and alkyl acrylate with an alkyl group having a carbon number of 2 to 20.

Typical examples of a monomer other than the alkyl methacrylate with an alkyl group having a carbon number of 6 to 22 and the alkyl acrylate with an alkyl group having a carbon number of 2 to 20 as described above include: alkyl methacrylate with an alkyl group having a carbon number of 5 or less than 5, such as methyl methacrylate, ethyl methacrylate and butyl methacrylate; (meth)acrylic acids; 1-vinyl-2-pyrrolidone; vinyl acetate; and hydroxyalkyl (meth) acrylate. When such monomer is contained as a monomer serving as a raw material for an acrylic adhesive, it becomes practicable to adjust the preservation stability of the DMAEs contained in the plaster layer and the adhesive strength of the transdermal patch. The (meth)acrylic acid refers to methacrylic acid or acrylic acid.

Further, as far as the effect of the present invention is not spoiled, the acrylic adhesive described above may be prepared by copolymerizing monomers containing a multifunctional monomer. Such multifunctional monomer is the one that has, in a single molecule, two or more functional groups exhibiting a radical polymerization, such as a vinyl group and an allyl group. Typical examples include divinyl benzene, methylene bisacrylamide, ethylene glycol di(meth) acrylate, hexanediol di(meth)acrylate, polyethylene glycol di(meth)acrylate and trimethylolpropane tri(meth)acrylate. In this way, when the polyfunctional monomer is added to the monomer serving as a raw material for the acrylic adhesive, the internal cohesion of the acrylic adhesive increases, so that an adhesive transfer to the skin can become less likely to occur when the transdermal patch is removed away from the skin. The (meth)acrylate refers to methacrylate or acrylate.

Further, as far as the preservation stability of the DMAEs is not spoiled, a crosslinking agent other than the above-mentioned polyfunctional monomer may be added to the monomer serving as a raw material for the acrylic adhesive, and typical examples of the crosslinking agents include an epoxy compound, a polyisocyanate compound, a metal chelate compound and a metal alkoxide compound. In this way, when the crosslinking agent is added to the monomer serving as a raw material for the acrylic adhesive, the internal cohesion of the acrylic adhesive increases, so that the adhesive transfer can become less likely to occur when the transdermal patch is removed away from the skin.

And, a method for polymerizing the above-described acrylic adhesive may be followed in accordance with a conventionally known method; for example, in the presence of a polymerization initiator, the monomers as described above are contained and a solution polymerization is carried out to accomplish the polymerization. Specifically, respectively prescribed amounts of alkyl methacrylate with an alkyl group having a carbon number of 6 to 22, alkyl acrylate with an alkyl group having a carbon number of 2 to 20, polymerization initiator and crosslinking agent added optionally are supplied, in joint with a polymerization solvent, to a reactor being equipped with a stirring device and a reflux condenser for an evaporated solvent, and heated for 4 to 48 hours at a temperature of about 80°C to subject the above-mentioned monomers to a radical polymerization reaction. Such polymerization reaction is preferably carried out in a nitrogen gas atmosphere.

Exemplary agents to be used as the polymerization initiator mentioned above are a polymerization initiator of an azobis type such as 2, 2'- azobisisobutyronitrile (AIBN), 1, 1'-azobis- (cyclohexane-1-carbonitrile), 2, 2'-azobis-(2, 4'-dimethylvaleronitrile); and a polymerization initiator of a peroxide type such as benzoyl peroxide (BPO), lauroyl peroxide (LPO) and di-tertiary-butyl peroxide. Typical examples of the above-mentioned polymerization solvent include ethyl acetate and toluene.

The transdermal patch of the present invention is required to exhibit high transdermal absorbency, because the plaster layer is applied to the skin to allow the DMAEs contained in the plaster layer to be absorbed through the skin. In order to improve such transdermal absorbency, it is preferable to increase the solvency and diffusibility of the DMAEs in the plaster layer, but when the solvency of the DMAEs is too high, the preservation stability of the DMAEs may sometimes be spoiled. Further, when the solvency of the DMAEs contained in the plaster layer is too high, the DMAEs, without being diffused in the plaster layer, may sometimes not be well distributed from the plaster layer to the skin, resulting in the decrease in the transdermal absorbency of the DMAEs.

In the transdermal patch of the present invention, therefore, the solvency of the DMAEs contained in the plaster layer is suitably improved by compounding, in the plaster layer, the fatty acid ester having a specific range of solvency of the DMAEs, the fatty acid ester being prepared by dehydro-condensing saturated fatty acid with an alkyl group having a carbon number of 10 to 20 and saturated aliphatic monohydric alcohol with an alkyl group having a carbon number of 2 to 20, without spoiling the preservation stability of the DMAEs contained in the plaster layer. Further, the fatty acid ester exhibits excellent compatibility with the acrylic adhesive, so that the acrylic adhesive is plasticized to increase the diffusibility of the DMAEs contained in the plaster layer, and thus affording a sufficient level of transdermal absorbency to show a drug effect.

Further, the fatty acid ester mentioned above has an effect of improving cutaneous permeability of the DMAEs by softening a stratum corneum of the skin or by increasing hydration of the skin and also has an effect of acting as a carrier for delivering the DMAEs inside the skin, so that the transdermal absorbency of the DMAEs can be improved to a large extent.

The saturated fatty acid described above is monohydric carboxylic acid expressed in a formula of R-COOH (R representing an alkyl group). When a carbon number of the alkyl group R is small, the compatibility obtained between the fatty acid ester and the acrylic adhesive decreases, the plasticization of the acrylic adhesive becomes insufficient, the diffusibility of DMAEs contained in the plaster layer decreases, and the transdermal absorbency of the DMAEs decreases. When a carbon number of the alkyl group R is large, the solvency of the DMAEs decreases, the plasticization of the acrylic adhesive becomes insufficient, the diffusibility of the DMAEs contained in the plaster layer decreases, and the transdermal absorbency of the DMAEs decreases. Therefore, a carbon number of the alkyl group R is limited to 10 to 20, and preferably 11 to 16.

The saturated aliphatic monohydric alcohol described above is monohydric alcohol expressed in a formula of R'-OH (R' representing an alkyl group). When a carbon number of the alkyl group R' is small, the compatibility decreases which is obtained between the fatty acid ester and the acrylic adhesive, the plasticization of the acrylic adhesive becomes insufficient, the diffusibility of DMAEs contained in the plaster layer decreases, and the transdermal absorbency of the DMAEs decreases. When a carbon number of the alkyl group R' is large, the solvency of the DMAEs contained in the plaster layer decreases, the plasticization of the acrylic adhesive becomes insufficient, the diffusibility of the DMAEs contained in the plaster layer decreases, and the transdermal absorbency of the DMAEs decreases. Therefore, a carbon number of the alkyl group R' is limited to 2 to 20, and preferably 2 to 14.

Such fatty acid ester is not specifically limited, but typical examples include hexyl laurate, isopropyl myristate, myristyl myristate, octyldodecyl myristate and isopropyl palmitate; and the isopropyl myristate is preferred.

The content of the fatty acid ester in the plaster layer described above is limited to 3 to 40% by weight, preferably 5 to 40% by weight, and more preferably 10 to 35% by weight. This is because, when the content of the fatty acid ester in the plaster layer is small, the plasticization of the acrylic adhesive becomes insufficient, the diffusibility of the DMAEs in the plaster layer decreases, and the transdermal absorbency of the DMAEs decreases; and when the content of the fatty acid ester in the plaster layer is large, the acrylic adhesive becomes excessively plasticized, and an adhesive transfer occurs when the transdermal patch is removed away from the skin.

According to the present invention, solubility of the DMAEs contained in the plaster layer, at 25°C with respect to the fatty acid ester, is limited to 0.05 to 5 g, preferably 0.1 to 5 g, more preferably 0.1 to 3 g, and most preferably 0.1 to 1 g. When the solubility of the DMAEs at 25°C with respect to the fatty acid ester is small, the amount of transdermal absorption becomes small; and when an area of the transdermal patch is not made large, the blood concentration of the DMAE cannot be increased to a desired range. This is because, when the solubility of the DMAEs at 25°C with respect to the fatty acid ester is large, the preservation stability of the DMAEs contained in the plaster layer decreases. The solubility of the DMAEs contained in the plaster layer, at 25°C with respect to the fatty acid ester, refers to the maximum amount in grams of the DMAEs that is soluble at 25°C in 100 g of the fatty acid ester.

An explanation shall now be made below about a method of measuring the above-described solubility of the DMAEs at 25°C with respect to the fatty acid ester. An excess amount of DMAEs is added to the fatty acid ester, a weight $W_0$ (g) of the fatty acid ester being measured in advance; a solution temperature is set at 50°C to keep the warmth for one hour or more than one hour, and then a shaking operation is continued in a water bath of 25°C for 10 minutes by means of supersonic waves to prepare a DMAEs saturated solution. Next, the DMAEs saturated solution, after being arranged to stand still for 24 hours at 25°C, is centrifuged at a speed of 3000 r.p.m., and a certain amount of clear supernatant solution of the DMAEs saturated solution is collected. Then, such collected clear supernatant solution is subjected to HPLC determination to determine a weight $W_1$ (g) of the DMAEs dissolved in the DMAEs saturated solution, and solubility of the DMAEs with respect to the fatty acid ester is figured out by using the following equation (1).

$$\text{Solubility of DMAEs (g)} = (W_1/W_0) \times 100 \qquad \text{Equation (1)}$$

As far as the effect of the present invention is not spoiled, a plasticizer, a solubilizing agent, an absorption promoting agent; a stabilizer, a filler, etc. may be added to the plaster layer described above.

The plasticizer mentioned above is added for the purpose of improving the adhesive strength of the transdermal patch and the diffusibility of the DMAEs in the plaster layer. Typical examples of such plasticizer include hydrocarbon such as liquid paraffin; ester resulting from reaction between aliphatic carboxylic acid and monohydric or polyhydric alcohol, such as glycerin monolaurate and diethyl sebacate; aliphatic alcohol such as myristyl alcohol and octyldodecanol; and oil and fat derived from natural organisms such as lanolin and olive oil. An addition of 1 to 10% by weight to the plaster layer will suffice.

The solubilizing agent mentioned above is added for the purpose of increasing solubility of the DMAEs in the plaster layer. Typical examples of such sulubilizing agent include polyhydric alcohol such as polyethylene glycol, propylene glycol and glycerin, and esters such as triacetin. An addition of 1 to 10% by weight in the plaster layer will suffice.

The absorption promoting agent mentioned above is used for acting on the skin to increase the cutaneous permeability, and use is made of a type for softening the stratum corneum or a type for increasing the hydration of the stratum corneum. Typical examples of such absorption promoting agent include a surfactant, etc. such as polysorbate, diethanolamide laurate, lauroyl sarcosine, polyoxyethylene alkyl ether and polyoxyethylene alkylamine; and an addition of 0.05 to 10% by weight in the plaster layer will suffice.

The stabilizer mentioned above is added for the purpose of inhibiting an oxidation or a decomposition of the DMAEs. Typical examples of such stabilizer include: an antioxidant such as butyl hydroxy toluene and sorbic acid; cyclodextrin; and ethylenediamine tetraacetic acid. An addition of 0.05 to 10% by weight in the plaster layer will suffice.

The filler mentioned above is added to adjust the adhesive strength of the transdermal patch or the transdermal absorbency of the DMAEs. Typical examples of such filler include: calcium carbonate, organometallic salt (such as magnesium stearate); an inorganic filler (such as anhydrous silicic acid and titanium oxide); a cellulose derivative (such lactose, crystalline cellulose, ethyl cellulose and low substituted hydroxypropylcellulose); vinylpyrrolidone; and a polymer resulting from the monomer of (meth)acrylic acid and (meth)acrylic derivatives. Particularly preferred are the anhydrous silicic acid, the low substituted hydroxypropylcellulose and the crosslinked polyvinyl pyrrolidone in that they do not affect the transdermal absorbency and preservation stability of the DMAEs and the adhesive properties can be well adjusted. An addition of 1 to 15% by weight of the filler in the plaster layer will suffice.

Typical examples of the low substituted hydroxypropylcellulose include low substituted hydroxypropylether of cellulose (refer to USP and Japanese Pharmaceutical Excipients) and low substituted hydroxypropylcellulose containing 5 to 16% of hydroxypropyl group in a dry state (refer to Japanese Pharmaceutical Excipients).

The low substituted hydroxypropylcellulose is commercially available from Shin-Etsu Chemical Co., Ltd., under the trade names of LH-20, LH-30, LH-11, LH-21, LH-31, LH-22 and LH-32.

The crosslinked polyvinyl pyrrolidone is commercially available from ISP Japan Ltd. under the trade names of Polyplasdone XL, Polyplasdone XL-10 and Polyplasdone INF-10, and also commercially available are Kollidon CL, Kollidon CL-M and Kollidon CL-SF from BSF Japan Ltd.

A thickness of the plaster layer described above is preferably 10 to 250 μm and more preferably 20 to 200 μm. When the thickness of the plaster layer is smaller than 10 μm, the DMAEs of the amount required in obtaining the desired blood concentration of the DMAE cannot sometimes be contained in the plaster layer. When the thickness of the plaster layer is larger than 250 μm, a problem may occur in that the plaster layer is liable to ooze out of the transdermal patch while the transdermal patch is in storage or when the transdermal patch is applied to the skin; in that a feeling becomes worsened when the transdermal patch is applied to the skin; or in that producibility decreases due to a time-consuming removal of the solvent when the transdermal patch is produced by coating the solvent as described below.

The support being integrally laminated with the plaster layer described above to constitute the transdermal patch of the present invention is meant for inhibiting a loss of the DMAEs contained in the plaster layer and also for protecting the plaster layer; and therefore the support is required to have a sufficient strength to afford a self-supporting ability to the transdermal patch and also to have sufficient flexibility to provide a good feeling when the patch is applied to the skin.

A material of such support is not specifically limited, and typical examples include a resin sheet, a foamed resin sheet, an unwoven fabric, a woven fabric, a knitted fabric and an aluminum sheet; the material may be structured either in a monolayer or in integrally laminated multilayers.

Typical examples of a resin constituting the resin sheet mentioned above include cellulose acetate, ethyl cellulose, rayon, polyethylene terephthalate, plasticized vinyl acetate-vinyl chloride copolymer, nylon, ethylene-vinyl acetate copolymer, plasticized polyvinyl chloride, polyurethane, polyethylene, polypropylene and polyvinylidene chloride; and the polyethylene terephthalate is referred.

A material of the support, from the point of view of its flexibility and an effect of inhibiting a loss of the DMAEs, is preferably a composite material in which the polyethylene terephthalate sheet is integrally laminated with the unwoven fabric or other soft resin sheet, and more preferably a composite material in which the polyethylene terephthalate sheet is integrally laminated with the unwoven fabric. Typical examples of a raw material constituting the unwoven fabric mentioned above include polyethylene, polypropylene, ethylene-vinyl acetate copolymer, ethylene-methyl (meth)acrylate copolymer, nylon, polyester, vinylon, SIS copolymer, SEBS copolymer, rayon and cotton; and the polyester is preferred. The raw material may be used either alone or in combination of two or more kinds.

In the case when the support mentioned above is of an integral lamination of the polyethylene terephthalate sheet and the unwoven fabric, if the polyethylene terephthalate sheet is thin, the sheet may not be uniformly bonded at the time of the integral lamination with the unwoven fabric, an obtained support may be short of a strength, or a pinhole occurred may cause a delamination in the interface between the polyethylene terephthalate sheet and the unwoven fabric. When the polyethylene terephthalate sheet is thick, the obtained support may be short of flexibility, conformity to the skin may decrease when the transdermal patch is applied to the skin, and the feeling may be worsened when the patch is applied to the skin. Therefore, a thickness of the polyethylene terephthalate sheet is preferably 5 to 200 μm.

Further, in the case when the support mentioned above is of an integral lamination of the polyethylene terephthalate sheet and the unwoven fabric, if the unwoven fabric is thin, its bondage with the polyethylene terephthalate sheet may become worsened, or the support may be short of a strength; on the other hand, when the unwoven fabric is thick, flexibility of the support may decrease. Hence, a thickness of the unwoven fabric is preferably 10 to 300 μm.

A method of producing the support in an integral lamination of the polyethylene terephthalate sheet and the unwoven fabric is not specifically limited; and exemplary methods include an integrally laminating process using a binder and a thermal bonding process. At the time of producing the support, a strength or a texture of the support can be adjusted by adding a binder or by partially, thermally bonding the polyethylene terephthalate sheet and the unwoven fabric.

In the transdermal patch of the present invention, for the purpose of inhibiting a loss of the DMAEs contained in the plaster layer and protecting the plaster layer, it is preferred that a release liner be releasably laminated integrally on the surface of the plaster layer of the transdermal patch.

Typical examples of the release liner mentioned above include a resin film (such as made of polyethylene terephthalate, polyethylene, polypropylene, polyvinyl chloride and polyvinylidene chloride) and paper, and it is preferred that the film sheet or paper be release-coated on the surface facing the plaster layer. The release liner described above may be either of a monolayer or of multilayers.

The release liner described above, for the purpose of improving its barrier property, may be provided with a layer such as an aluminum foil and aluminum deposition. Further, when the release liner is paper, for the purpose of improving its barrier property, the paper may be impregnated with a resin such as polyvinyl alcohol.

Next, an explanation shall be made for a method for producing the transdermal patch of the present invention. The method for producing the patch described above is not specifically limited, but typical methods include: a method in which the DMAEs, the acrylic adhesive, the fatty acid ester and the additive (optionally added) are added to the solution such as ethyl acetate; such mixture is stirred to become uniform in obtaining a solution to be used as the plaster layer; such solution is coated on one surface of the support in a process such as a solvent coating process and a hot-melt coating process; the coated solution is dried to integrally laminate the plaster layer on the one surface of the support; and optionally the release liner is applied, for an integral lamination, on to the plaster layer in a manner that the release-coated surface of the release liner faces the plaster layer, and an alternative method in which, according to the coating process described above, the solution to be used for the plaster layer is coated on the release-coated surface of the release liner; the coated solution is dried to form the plaster layer on the release liner; and the support is integrally laminated on to the plaster layer.

Effect of the Invention

In the transdermal patch of the present invention, since the specifically structured acrylic adhesive and fatty acid ester are contained in the plaster layer, the solvency and diffusibility of the DMAEs are improved without spoiling the preservation stability of the DMAEs contained in the plaster layer, and a sufficient amount of DMAEs is contained in the plaster layer in a diffused state.

Further, in the transdermal patch of the present invention, since the cutaneous permeability of the DMAEs is improved by the specifically structured fatty acid ester described above, and the above-described fatty acid ester itself serves as a carrier for delivering the DMAEs inside the skin, the patch exhibits an excellent transdermal absorbency of the DMAEs. Therefore, the transdermal patch described above is advantageously used as a therapy drug for an essential hypotension and an orthostatic hypotension and also as an administration drug or mecine of the DMAEs which will, in the future, be expected to be a medicine applied for treatment of abdominal pressure-induced incontinence.

Further, in the transdermal patch described above, since the specifically structured acrylic adhesive and fatty acid ester are contained in the plaster layer, the preservation stability of the DMAEs contained in the plaster layer is excellent, and the DMAEs are almost unlikely to be decomposed. Therefore, when the patch is applied to the skin, the stipulation on the skin almost rarely occurs which may be caused by the degradation product in the DMAEs.

Additionally, in the transdermal patch described above, since a suitable adhesive strength is possessed by the patch which is not likely to be unexpectedly peeled off the skin while being applied and also the adhesive transfer to the skin is rarely caused when the patch is removed away from the skin, the patch is advantageously used for administering the DMAEs.

Brief Description of the Drawings

FIG. 1 is a graph depicting the results of the release tests performed in the inventive examples 1 to 4 and the comparative example 1;
FIG. 2 is a graph depicting the results of the release tests performed in the inventive example 13 and the comparative example 13;
FIG. 3 is a graph depicting the results of the release tests performed in the comparative examples 15 and 18;
FIG. 4 is a graph depicting the results of the release tests performed in the comparative examples 16 and 19;
FIG. 5 is a graph depicting the results of the release tests performed in the comparative examples 17 and 20;
FIG. 6 is a graph depicting the results of the permeability tests performed in the inventive examples 4 and 5 and the comparative examples 9 and 11;
FIG. 7 is a graph depicting the results of the permeability tests performed in the inventive example 13 and the comparative example 13;
FIG. 8 is a graph depicting the results of the permeability tests performed in the inventive example 14 and the comparative example 14;
FIG. 9 is a graph depicting the results of the permeability tests performed in the comparative examples 15 and 18;
FIG. 10 is a graph depicting the results of the permeability tests performed in the comparative examples 16 and 19; and
FIG. 11 is a graph depicting the results of the permeability tests of the DMAEs performed in the comparative examples 17 and 20.

Best Mode for Carrying Out the Invention

First, in the alkyl methacrylate, the alkyl acrylate and other monomers, solvency and residual rate (% by weight) of DMAE, tulobuterol (TB), indomethacin (IMT) and isosorbide dinitrate (ISDN) (hereinafter, these four kinds of compounds shall be collectively referred to as "active substance") were evaluated in the under-mentioned procedure, results of which are illustrated in Table 1.

(Solvency of the active substance)

As illustrated in Table 1, 100 parts by weight of monomer were sufficiently mixed with 3 parts by weight of any one compound of the DMAE, the TB, the IMT and the ISDN to prepare a solution of active substance, and a visual inspection was conducted for a state of the active substance dissolved in the solution of active substance. Then, after the solution of active substance was preserved at 25°C for 7 days, a visual inspection was again conducted for a state of the active substance dissolved in the solution of active substance to evaluate solvency of the active substance in accordance with the following standard.

Excellent: The active substance was not found to have been precipitated in the solution of active substance both immediately after the preparation and after the preservation for 7 days.

Good : The active substance was found to have been precipitated in the solution of active substance immediately after the preparation, but the active substance was not found to have been precipitated in the solution of active substance after the preservation for 7 days.

Poor : The active substance was found to have been precipitated in the solution of active substance both immediately after the preparation and after the preservation for 7 days.

(Residual rate of the active substance)

After arrangement of the solution of active substance immediately after being prepared in the above-described procedure and also such solution of active substance, being prepared in the above-described procedure, having been preserved at a temperature of 25°C for 7 days, a weight $W_3$ (g) of the active substance contained in these solutions of active substance was determined by means of the HPLC; and a residual rate (% by weight) with respect to the amount of addition ($w_2$ (g)) of the active substance was figured out, based on the following equation (2).

$$\text{Residual Rate of Active Substance (\% by weight)}=(W_3/W_2)\times 100 \qquad \text{Equation (2)}$$

The "N.D." appearing in Table 1 indicates that the residual amount of active substance in the solution of active substance was equal to or less than the detection limit by means of the HPLC, and an evaluation was not made of the solution of DMAE after the preservation for 7 days with regard to the ethyl acrylate in which the residual amount of DMAE in the DMAE solution immediately after the preparation was equal to or less than the detection limit by means of the HPLC.

Subsequently, an evaluation was made of preservation stability, solvency and solubility of the DMAE contained in the fatty acid ester and the additive which were used for preparing the transdermal patch, the results of which are illustrated in Table 2.

(Preservation stability of DMAE)

After 3 parts by weight of the DMAE and 100 parts by weight of the compound indicated in Table 2 were sufficiently mixed, such mixture was contained in a sealed vessel and preserved at a temperature of 60°C for 10 days. Then, a visual inspection was conducted for a color of the mixture after the preservation; and the state without any color change after the preservation as compared with the color prior to the preservation was evaluated to be "Excellent", while the state with a color change was evaluated to be "Poor".

Further, a weight $W_5$ (g) of the DMAE remaining undecomposed in the mixture after the preservation as described above was determined by means of the HPLC, and the residual rate (% by weight) of the DMAE with respect to the added amount $W_4$ (g) of DMAE was figured out on the basis of the following equation (3).

$$\text{Residual Rate of DMAE (\% by weight)}=(W_5/W_4)\times 100 \qquad \text{Equation (3)}$$

The "N.D." appearing in Table 2 indicates that the amount of the DMAE in the mixture was equal to or less than the detection limit by means of the HPLC. Further, an evaluation was not made of the residual rate of the DMAE with regard to isostearic acid, oleic acid and polyoxyethylene (2) lauryl ether in which a color change was found by a visual inspection.

(Solvency of DMAE)

The DMAE solution was prepared which was composed of 50 parts by weight of DMAE and 950 parts by weight of compound indicated as a solvent in Table 2. Next, the DMAE solution was kept warm at 50°C for 2 hours, and then was shaken for 10 minutes by means of ultrasonic waves in a water bath at 25°C. Then, after the DMAE solution was transferred into a centrifuge tube and kept still at 25°C for 24 hours, the DMAE solution was centrifuged at a speed of 3000 r.p.m. by using a centrifugal separator. When a crystal of DMAE was precipitated in the bottom of the centrifuge tube, the solvency was evaluated to be "excellent", and when a crystal of DMAE was not precipitated, the solvency was evaluated to be "poor".

(Solubility of DMAE)

Then, with regard to the one in which the crystal of the DMAE was precipitated in the above evaluation of "Solvency of DMAE", a certain amount of clear supernatant solution of the DMAE solution after having been centrifuged was collected and such collected clear supernatant solution was subjected to the HPLC determination to determine a weight $W_7$ (g) of the DMAE dissolved in the DMAE solution mentioned above; and solubility (g) of DMAE at 25°C with respect to the compound serving as the solvent was figured out, based on the following equation (4).

$$\text{Solubility of DMAE (g)} = (W_7/W_6) \times 100 \qquad \text{Equation (4)}$$

($W_6$: a weight (g) of the compound serving as the solvent in the DMAE solution)

Next, acrylic adhesives A to I used as an adhesive for the transdermal patch of the present invention were prepared in the following procedure, and transdermal patches were produced as illustrated in the inventive examples 1 to 20 and the comparative examples 1 to 20.

(Preparation of acrylic adhesive A) (according to the invention)

A reaction solution composed of 2,286 parts by weight (12.56% by weight) of dodecyl methacrylate, 14,256 parts by weight (78.34% by weight) of 2-ethylhexyl methacrylate, 1,656 parts by weight (9.10% by weight) of 2-ethylhexyl acrylate and 8,500 parts by weight of ethyl acetate was charged into a 40-liter polymerizing apparatus, in which a nitrogen atmosphere was maintained at 80°C. Then, in order to obtain a solution of acrylic adhesive A with a content of 35% by weight of acrylic adhesive A, a polymerization was performed by adding to the above-mentioned reaction solution, for 24 hours, a solution of polymerization initiator in which 16 parts by weight of benzoyl peroxide were dissolved in 1,500 parts by weight of cyclohexane, and further by adding ethyl acetate after such polymerization.

(Preparation of acrylic adhesive B) (not the invention)

A reaction solution composed of 100 parts by weight of 2-ethylhexyl acrylate, 80 parts by weight of ethyl acrylate, 20 parts by weight of 1-vinyl-2- pyrrolidone and 200 parts by weight of ethyl acetate was introduced into a separable flask, in which a nitrogen atmosphere was maintained at 80°C. Then, in order to obtain a solution of acrylic adhesive B with a content of 32% by weight of acrylic adhesive B, a polymerization was performed by adding to the reaction solution, for 27 hours, a solution of polymerization initiator in which 1 part by weight of benzoyl peroxide was dissolved in 100 parts by weight of cyclohexane, and further by adding ethyl acetate after such polymerization.

(Preparation of acrylic adhesive C) (not the invention)

A reaction solution composed of 150 parts by weight of 2-ethylhexyl acrylate, 50 parts by weight of 1-vinyl-2-pyrrolidone and 200 parts by weight of ethyl acetate was introduced into a separable flask, in which a nitrogen atmosphere was maintained at 80°C. Then, in order to obtain a solution of acrylic adhesive C with a content of 32% by weight of acrylic adhesive C, a polymerization was performed by adding to the reaction solution, for 27 hours, a solution of polymerization initiator in which 1 part by weight of benzoyl peroxide was dissolved in 100 parts by weight of cyclohexane, and further by adding ethyl acetate after such polymerization.

(Preparation of acrylic adhesive D) (according to the invention)

A reaction solution composed of 20 parts by weight of dodecyl methacrylate, 30 parts by weight of hexyl methacrylate, 50 parts by weight of butyl acrylate and 40 parts by weight of ethyl acetate was introduced into a separable flask, in which a nitrogen atmosphere was maintained at 80°C. Then, in order to obtain a solution of acrylic adhesive D with a content of 35% by weight of acrylic adhesive D, a polymerization was performed by adding to the reaction solution, for 10 hours, a solution of polymerization initiator in which 1 part by weight of benzoyl peroxide was dissolved in 100 parts by weight of cyclohexane, and further by adding ethyl acetate after such polymerization.

(Preparation of acrylic adhesive E) (not the invention)

A reaction solution composed of 15 parts by weight of dodecyl methacrylate, 85 parts by weight of 2-ethylhexyl methacrylate and 50 parts by weight of ethyl acetate was introduced into a separable flask, in which a nitrogen atmosphere was maintained at 80°C. Then, in order to obtain a solution of acrylic adhesive E with a content of 32% by weight of acrylic adhesive E, a polymerization was performed by adding to the reaction solution, for 10 hours, a solution of polymerization initiator in which 0.5 parts by weight of azobisisobutyronitrile were dissolved in 50 parts by weight of ethyl acetate, and further by adding ethyl acetate after such polymerization.

(Preparation of acrylic adhesive F) (according to the invention)

A reaction solution composed of 75 parts by weight of 2-ethylhexyl methacrylate, 15 parts by weight of butyl methacrylate, 10 parts by weight of 2-ethylhexyl acrylate and 50 parts by weight of ethyl acetate was introduced into a separable flask, in which a nitrogen atmosphere was maintained at 80°C. Then, in order to obtain a solution of acrylic adhesive F with a content of 33% by weight of acrylic adhesive F, a polymerization was performed by adding to the reaction solution, for 10 hours, a solution of polymerization initiator in which 0.5 parts by weight of azobisisobutyronitrile were dissolved in 50 parts by weight of ethyl acetate, and further by adding ethyl acetate after such polymerization.

(Preparation of acrylic adhesive G) (not the invention)

A reaction solution composed of 75 parts by weight of 2-ethylhexyl methacrylate, 25 parts by weight (50% by weight) of 1-vinyl-2-pyrrolidone and 50 parts by weight of ethyl acetate was introduced into a separable flask, in which a nitrogen atmosphere was maintained at 80°C. Then, in order to obtain a solution of acrylic adhesive G with a content of 35% by weight of acrylic adhesive G, a polymerization was performed by adding to the reaction solution, for 10 hours, a solution of polymerization initiator in which 0.5 parts by weight of azobisisobutyronitrile were dissolved in 50 parts by weight of ethyl acetate, and further by adding ethyl acetate after such polymerization.

(Preparation of acrylic adhesive H) (not the invention)

A reaction solution composed of 95 parts by weight of 2-ethylhexyl acrylate, 5 parts by weight of acrylic acid and 50 parts by weight of ethyl acetate was introduced into a separable flask, in which a nitrogen atmosphere was maintained at 80°C. Then, in order to obtain a solution of acrylic adhesive H with a content of 35% by weight of acrylic adhesive H, a polymerization was performed by adding to the reaction solution, for 10 hours, a solution of polymerization initiator in which 0.5 parts by weight of azobisisobutyronitrile were dissolved in 50 parts by weight of ethyl acetate, and further by adding ethyl acetate after such polymerization.

(Preparation of acrylic adhesive I) (according to the invention)

A reaction solution composed of 30 parts by weight of 2-ethylhexyl methacrylate, 70 parts by weight of 2-ethylhexyl acrylate and 100 parts by weight of ethyl acetate was introduced into a separable flask, in which a nitrogen atmosphere was maintained at 80°C. Then, in order to obtain a solution of acrylic adhesive I with a content of 30% by weight of acrylic adhesive I, a polymerization was performed by adding to the reaction solution, for 10 hours, a solution of polymerization initiator in which 0.5 parts by weight of azobisisobutyronitrile were dissolved in 100 parts by weight of ethyl acetate, and further by adding ethyl acetate after such polymerization.

(Inventive Examples 1 to 20 and Comparative Examples 1 to 20)

A solution used for the plaster layer was prepared by containing a DMAE, a solution of acrylic adhesive, fatty acid ester and an additive so that a weight composition of the DMAE, the acrylic adhesive, the fatty acid ester and the additive all in the plaster layer have a ratio as indicated respectively in Tables 3 to 6; and ethyl acetate was added so that the concentration of a solid content becomes 22% by weight; and then such mixture was mixed to become homogeneous. In the column for the fatty acid ester as indicated respectively in Tables 3 to 6, a carbon number of an alkyl group respectively for the saturated fatty acid and the saturated aliphatic monohydric alcohol having served as a raw material for each compound was stated one by one in the parentheses located on the right side of the compound.

To inform the source of supply for certain chemicals that were used in the experiment, the crosslinked polyvinyl pyrrolidone is commercially available under the trade name of "Polyplasdone INF-10" from ISP Japan Ltd.; the low substituted hydroxypropylcellulose is commercially available under the trade name of "LH-31" from Shin-Etsu Chemical Co., Ltd.; the light anhydrous silicic acid is commercially available under the trade number of "Aerosil 200" from Nippon Aerosil Co., Ltd.; the ethyl cellulose is commercially available under the trade number of "Ethocel 100CUPS" from Nisshin Kasei Co., Ltd.; the polyvinyl acetal diethylaminoacetate is commercially available under the trade name of "AEA Sankyo" from Sankyo Lifetech Co., Ltd.; the carboxymethyl cellulose is available under the trade name of "NS-300" from Gotoku Chemical Company Ltd.; the methacrylate copolymer is commercially available under the trade name of "EUDRAGID L100" from Higuchi Inc.; and the hydroxypropylmethylcellulose phthalate is commercially available under the trade name of "HP-55S" from Shin-Etsu Chemical Co., Ltd.

Next, a 38-μm thick, siliconized, release coated polyethylene terephthalate film was prepared, the solution used for the plaster layer described above was coated on a siliconized, release coated surface of the polyethylene terephthalate film, and such film was dried at 60°C for 30 minutes; thus a laminated layer was produced in which the plaster layer with a respective thickness as indicated in Tables 3 to 6 was formed on the siliconized, release coated surface of the polyethylene terephthalate film.

Then, a support was prepared in which a 12-μm thick polyethylene terephthalate film was thermally bonded with a polyester unwonven fabric having a thickness of about 160 μm and a grammage of 40 $g/m^2$; the polyethylene terephthalate film side of the support was faced to and laminated with the plaster layer of the laminated layer mentioned above to produce a transdermal patch by integrally laminating the plaster layer of the lamination layer on the support.

Next, the transdermal patch thus obtained was evaluated in the following manner. Specifically, an evaluation was made of surface appearances based on homogeneity of the plaster layer at the time of production. Included in a stability test were an evaluation of the residual rate of the DMAE and a yellowing state. Tests for application of the patch on a rat and permeability were carried out to evaluate a drug absorption into the skin. A release test was conducted to evaluate a rate of drug release out of the plaster layer. An evaluation was made of a peeling state and an adhesive transfer to evaluate a plastering ability of the patch. These evaluation results are indicated in Tables 3 to 6. In Tables 3 to 6, however, when the patch was not subjected to an evaluation, a mark of "-" was given in a relevant column, or there was not given any relevant column for evaluation.

(Residual Rate of DMAE)

Cut out of the obtained transdermal patch were two test pieces respectively with an area of 3 $cm^2$. 5 mL of ethyle acetate-ethanol mixed solvent (a capacity ratio of 80:20 between the ethyl acetate and the ethanol) was added to one of the test pieces to extract a component out of the test piece. The ethyl acetate-ethanol extraction solution thus obtained was subjected to the HPLC determination to determine a DMAE content $W_8$ (μg) in the test piece.

Next, the other one of the test pieces was sealed in a package composed of a lamination of a polyester film and a polyacrylonitrile film, and preserved at 60°C for 20 days. Subsequently, 5 mL of ethyl acetate-ethanol mixed solvent (a capacity ratio of 80:20 between the ethyl acetate and the ethanol) was added to such test piece having been preserved for 20 days to extract a component out of the aforesaid test piece having been preserved for 20 days, and the ethyl acetate-ethanol extraction solution thus obtained was subjected to the HPLC determination to determine a DMAE content $W_9$ (μg) in the test piece having been preserved for 20 days.

Then, as obtained above, the DMAE content $W_8$ (μg) in the test piece having not been subjected to the 20-day preservation and the DMAE content $W_9$ (μg) in the test piece having been preserved for 20 days were assigned into the following equation (5) to figure out a residual rate (% by weight) of the DMAE in the test piece having been preserved for 20 days.

$$\text{Residual Rate (\% by weight) of DMAE} = (W_9/W_8) \times 100 \qquad \text{Equation (5)}$$

(Yellowing State)

Two test pieces respectively with an area of 3 $cm^2$ were cut out from the obtained transdermal patch. After one of the test pieces had been preserved at 60°C for 20 days, the test piece was placed on white paper. After the other one of the test pieces had been preserved at 4°C for 20 days, the test piece was placed on white paper. These test pieces placed side by side were visually inspected. When no distinct difference was found between the two test pieces, the test piece was evaluated to be "excellent". When a slight yellowing state was found on the test piece having been preserved at 60°C, this particular test piece was evaluated to be "good". When a distinct yellowing state was found on the test piece having been preserved at 60°C, such test piece was evaluated to be "poor".

(Surface Appearance)

At the time of producing the transdermal patch, a visual inspection was conducted for a state of the plaster layer before lamination with the support. Regardless whether a crystal was in a dissolved state or in a precipitated state, when the plaster layer was found to be formed substantially in homogeneity, the surface appearance was evaluated to be "excellent"; when the state of the surface was found to be slightly inhomogeneous and lack a smoothness, the surface appearance was evaluated to be "good"; and when the surface of the plaster layer was found to be remarkably inhomogeneous, the surface appearance was evaluated to be "poor".

(Test for application of the patch on a rat)

A test piece with an area of 3 $cm^2$ was cut out of the obtained transdermal patch, the test piece was applied on the skin of a rat's back (a Wistar rat, male, 7 weeks old), with its hairs on the back having been removed in advance, and the test piece was removed away from the skin after 24 hours. Then, the ethyl acetate-ethanol mixed solvent was added to the test piece having been removed away from the skin, a component of the plaster layer was extracted, the component was subjected to the HPLC determination to determine a DMAE residual content $W_{10}$ ($\mu$g) contained in the ethyl acetate-ethanol extraction solution mentioned above, and an amount of transdermal absorption ($\mu g/cm^2/24h$) was figured out by using the following equation (6).

$$\text{Amount of Transdermal Absorption } (\mu g/cm^2/24h) = (W_8 - W_{10})/3 \qquad \text{Equation (6)}$$

(Skin Stimulation)

In the above-described test for application of the patch on a rat, immediately after removal of the test piece away from the rat's back, a visual inspection was conducted for a state of the skin of the rat's back, and an evaluation was made of a skin stimulation according to the following standard.

Excellent: No erythema was found on the skin.
Poor : An erythema was found on the skin.

(Peeling State)

In the above-described test for application of the patch on a rat, an applied state of the test piece was inspected before removal of the test piece away from the rat's back. When no peeling of the test piece was found; the applied state was evaluated to be "excellent"; when peeling of 5% or less than 5% with respect to the applied area was found, the applied state was evaluated to be "good"; and when peeling of more than 5% with respect to the applied area was found, the applied state was evaluated to be "poor".

(Adhesive Transfer)

Further, in the above-described test for application of the patch on a rat, a finger touch was done directly on the skin of a rat's back after removal of the test piece away from the rat's back. When no stickiness was found on the skin, the state was evaluated to be "excellent"; when stickiness was found only on a part of a peripheral edge around the applied portion, the state was evaluated to be "good"; and when stickiness was found wholly along the peripheral edge around the applied portion, the state was evaluated to be "poor".

Owing to an unstable base line in an HPLC chromatogram for a stability test, the transdermal patches in the comparative examples 2 and 3, which were judged that accuracy was not obtainable in the test for application of the patch on a rat, were not subjected to the test for application of the patch on the rat (a determination of an amount of transdermal absorption, and an evaluation of a peeling state and an adhesive transfer). Further, for the same reason, the patches in the comparative examples 4 and 7 were actually subjected to the test for application of the patch on a rat, but the test was only for evaluation of the skin stimulation, the peeling state and the adhesive transfer.

(Release Test)

Planar, circular test pieces (an area: about 10 $cm^2$) with a diameter of 3.6 cm were cut out of the transdermal patches produced in the inventive examples 1 to 4 and 13 and the comparative examples 1, 13, and 15 to 20. The test pieces were respectively applied, by using a double coated adhesive tape, to a stainless steel plate with a diameter of 5 cm and a thickness of 3 mm. Here, such application was so arranged that the plaster layer of the test piece was exposed. As a test solution, 300 mL of water was supplied to and kept in a release testing apparatus, at 37°C. The test piece was immersed in the test solution such that the plaster layer faced upward, and the test solution was stirred by an impeller at a rate of 100 revolutions per minute.

At the lapse of 30, 60 and 120 minutes respectively after the test piece had been immersed in the test solution, a certain amount of test solution was collected to determine a DMAE concentration by means of the HPLC. Then, a release amount of DMAE was figured out which was obtained on the basis of the DMAE concentration and the amount of test solution at each lapse of time. Three test pieces each were prepared for each transdermal patch, and a value of arithmetic mean of the release amounts of DMAE obtained from each individual test piece was set to be a release amount. In figuring out the release amount, since the test solution had already be collected, a correction was made to the collected amount of the test solution. The results are indicated in FIGS. 1 to 5.

(Permeability Test)

Planar, circular test pieces (an applied area: 3.14 $cm^2$) with a diameter of 2 cm were cut out of the transdermal patches produced in the inventive examples 4, 5, 13 and 14 and the comparative examples 9, 11 and 13 to 20. Further, a skin excised from the back of a hairless mouse (male, 8 to 10 weeks old) was fixed to Franz diffusion cell, and a physiological saline solution as a receptor solution adjusted at pH of 7.2 was filled in the diffusion cell located at the lower end side of the skin. The test piece was applied on the top end of the skin and was kept at 37°C.

After 3, 6, 9, 21 and 24 hours respectively since the test pieces had been applied on the skin, the receptor solution at the underside of the skin was collected to determine a DMAE concentration by means of the HPLC. Three test pieces were prepared here for each transdermal patch. Then, a permeable amount of DMAE was figured out on the basis of the DMAE concentration and the amount of receptor solution at each lapse of time, the permeable amount of DMAE figured out for each test piece was subjected to the arithmetic mean at each lapse of time, and such value was set to be an accumulative amount of cutaneous permeation. When the accumulative amount of permeation was figured out, a correction was made to the collected amount of receptor solution because the receptor solution had been collected beforehand. The results are indicated in FIGS. 6 through 11.

Table 1

| Monomer | DMAE | | | TB | | | IMT | | | ISDN | | |
|---|---|---|---|---|---|---|---|---|---|---|---|---|
| | Solvency of DMAE | Residual Rate of DMAE (% by Weight) | | Solvency of TB | Residual Rate of TB (% by weight) | | Solvency of IMT | Residual Rate of IMT (% by weight) | | Solvency of ISDN | Residual Rate of ISDN (% by weight) | |
| | | Immediately after Preparation | after 7 Days | | Immediately after Preparation | after 7 Days | | Immediately after Preparation | after 7 days | | Immediately after Preparation | after 7 Days |
| Dodecyl Methacrylate | Poor | 100 | 99 | Good | 100 | 100 | Poor | 100 | 100 | Excellent | 100 | 100 |
| 2-Ethylhexyl Methacrylate | Poor | 99 | 99 | Excellent | 100 | 100 | Poor | 100 | 100 | Excellent | 100 | 99 |
| Butyl Methacrylate | Poor | 98 | 98 | Excellent | 100 | 100 | Poor | 100 | 99 | Excellent | 100 | 100 |
| 2-Ethylhexyl Acrylate | Poor | 39 | 11 | Excellent | 100 | 88 | Poor | 100 | 100 | Excellent | 100 | 100 |
| Ethyl Acrylate | Excellent | N.D. | - | Excellent | 100 | 70 | Poor | 100 | 99 | Excellent | 100 | 100 |
| Butyl Acrylate | Good | 27 | N.D. | Excellent | 100 | 81 | Poor | 100 | 100 | Excellent | 100 | 100 |
| Acrylic Acid | Excellent | 99 | 97 | Excellent | 100 | 96 | Excellent | 100 | 100 | Excellent | 100 | 100 |
| 1-Vinyl-2-Pyrrolidone | Poor | 100 | 100 | Excellent | 100 | 100 | Excellent | 100 | 100 | Excellent | 100 | 100 |

Table 2

| Compound | Preservation Stability of DMAE | | Solvency of DMAE | Solubility of DMAE (g) |
|---|---|---|---|---|
| | Test for Color Change | Residual Rate of DMAE (% by weight) | | |
| Isopropyl Myristate | Excellent | 100 | Excellent | 0.2 |
| Isopropyl Palmitate | Excellent | 91.1 | Excellent | 0.2 |
| Octyldodecyl Myristate | Excellent | 100 | Excellent | 0.05 |
| Hexyl Laurate | Excellent | 82.1 | Excellent | 0.2 |
| Diethyl Sebacate | Excellent | 60.6 | Excellent | 0.9 |
| Medium-chain Triglyceride | Excellent | 0.9 | Excellent | 0.3 |
| Triacetin | Excellent | N.D. | Poor | - |
| Isostearic Acid | Poor | - | Poor | - |
| Oleic Acid | Poor | - | Excellent | 3.7 |
| Polyoxyethylene (2) Lauryl Ether | Poor | - | Excellent | 3.7 |
| Monoethanolamine | Excellent | 94.5 | Poor | - |
| Liquid Paraffin | Excellent | 96.1 | Excellent | 0.02 |
| Squalane | Excellent | 100 | Excellent | 0.01 |
| Butylene Glycol | Excellent | 97.9 | Poor | - |
| Propylene Glycol | Excellent | 98.1 | Poor | - |
| Octanediol | Excellent | 97.0 | Poor | - |
| Oleyl Alcohol | Excellent | 19.9 | Excellent | 2.5 |

Table 3

| | | | Inventive Example 1 | Inventive Example 2 | Inventive Example 3 | Inventive Example 4 | Inventive Example 5 | Inventive Example 6 | Inventive Example 7 | Inventive Example 8 | Inventive Example 9 | Inventive Example 10 |
|---|---|---|---|---|---|---|---|---|---|---|---|---|
| CONSTRUCTION | DMAE (% by weight) | | 10 | 15 | 15 | 10 | 10 | 10 | 10 | 10 | 10 | 10 |
| | Acrylic Adhesive (% by weight) | A | 80 | 60 | 55 | 51 | 65 | 65 | 65 | 65 | — | — |
| | | D | — | — | — | — | — | — | — | — | 65 | — |
| | | F | — | — | — | — | — | — | — | — | — | 65 |
| | | I | — | — | — | — | — | — | — | — | — | — |
| | Fatty Acid Ester (% by weight) | Isopropyl Myristate (C13-C3) | 10 | 20 | 20 | 26 | 20 | — | — | — | 20 | 20 |
| | | Isopropyl Palmitate (C15-C3) | — | — | — | — | — | 20 | — | — | — | — |
| | | Hexyl Laurate (C11-C6) | — | — | — | — | — | — | 20 | — | — | — |
| | | Octyldodecyl Myristate (C13-C20) | — | — | — | — | — | — | — | 20 | — | — |
| | Additive (% by weight) | Low Substituted Hydroxypropyl Cellulose | — | 5 | — | — | 5 | 5 | 5 | 5 | 5 | 5 |
| | | Crosslinked Polyvinyl Pyrrolidone | — | — | 10 | — | — | — | — | — | — | — |
| | | Light Anhydrous Silicic Acid | — | — | — | 13 | — | — | — | — | — | — |
| | | Ethyl Cellulose | — | — | — | — | — | — | — | — | — | — |
| | | Polyvinyl Acetal Diethylaminoacetate | — | — | — | — | — | — | — | — | — | — |
| | | Carboxymethyl Cellulose | — | — | — | — | — | — | — | — | — | — |
| | | Methacrylate Copolymer | — | — | — | — | — | — | — | — | — | — |
| | | Hydroxypropylmethylcellulose Phthalate | — | — | — | — | — | — | — | — | — | — |
| | Thickness of Plaster Layer (μm) | | 50 | 50 | 70 | 100 | 70 | 70 | 70 | 70 | 70 | 70 |
| EVALUATION | Stability Test | Residual Rate of DMAE (% by weight) | 97 | 97 | 94 | 90 | 98 | 97 | 96 | 99 | 96 | 98 |
| | | Yellowing State | — | — | — | — | Excellent | — | — | — | — | — |
| | Surface Appearance | | — | — | — | — | Excellent | — | — | — | — | — |
| | Test for Application of the Patch on a Rat | Amount of Transdermal Absorption ($\mu g/cm^2/24h$) | 70 | 90 | 128 | 196 | 121 | 119 | 126 | 77 | 125 | 112 |
| | | Skin Stimulation | Excellent | Excellent | Excellent | Excellent | Excellent | Excellent | Excellent | Excellent | Excellent | Excellent |
| | | Peeling State | Excellent | Excellent | Excellent | Excellent | Excellent | Excellent | Excellent | Excellent | Excellent | Excellent |
| | | Adhesive Transfer | Excellent | Excellent | Excellent | Excellent | Excellent | Excellent | Excellent | Excellent | Excellent | Excellent |

Table 4

| | | | Inventive Example 11 | Inventive Example 12 | Inventive Example 13 | Inventive Example 14 | Inventive Example 15 | Inventive Example 16 | Inventive Example 17 | Inventive Example 18 | Inventive Example 19 | Inventive Example 20 |
|---|---|---|---|---|---|---|---|---|---|---|---|---|
| CONSTRUCTION | DMAE (% by weight) | | 10 | 30 | 5 | 5 | 10 | 10 | 10 | 15 | 15 | 15 |
| | Acrylic Adhesive (% by weight) | A | — | 60 | — | — | 70 | 60 | 60 | 60 | 60 | 60 |
| | | D | — | — | 85 | — | — | — | — | — | — | — |
| | | F | — | — | — | 85 | — | — | — | — | — | — |
| | | I | 65 | — | — | — | — | — | — | — | — | — |
| | Fatty Acid Ester (% by weight) | Isopropyl Myristate (C13-C3) | 20 | 10 | — | — | 20 | 20 | 20 | 20 | 20 | 20 |
| | | Isopropyl Palmitate (C15-C3) | — | — | 10 | 10 | — | — | — | — | — | — |
| | | Hexyl Laurate (C11-C6) | — | — | — | — | — | — | — | — | — | — |
| | | Octyldodecyl Myristate (C13-C20) | — | — | — | — | — | — | — | — | — | — |
| | Additive (% by weight) | Low Substituted Hydroxypropyl Cellulose | 5 | — | — | — | — | — | — | — | — | — |
| | | Crosslinked Polyvinyl Pyrrolidone | — | — | — | — | — | — | — | — | — | — |
| | | Light Anhydrous Silicic Acid | — | — | — | — | — | — | — | — | — | — |
| | | Ethyl Cellulose | — | — | — | — | — | 10 | — | — | — | — |
| | | Polyvinyl Acetal Diethylaminoacetate | — | — | — | — | — | — | 10 | — | — | — |
| | | Carboxymethyl Cellulose | — | — | — | — | — | — | — | 5 | — | — |
| | | Methacrylate Copolymer | — | — | — | — | — | — | — | — | 5 | — |
| | | Hydroxypropylmethylcellulose Phthalate | — | — | — | — | — | — | — | — | — | 5 |
| | Thickness of Plaster Layer (μm) | | 70 | 70 | 70 | 70 | 70 | 70 | 70 | 70 | 70 | 70 |
| EVALUATION | Stability Test | Residual Rate of DMAE (% by weight) | 94 | 99 | — | — | 98 | 94 | 95 | 91 | 88 | 83 |
| | | Yellowing State | — | — | — | — | Excellent | Excellent | Excellent | Good | Good | Poor |
| | Surface Appearance | | — | — | — | — | Excellent | Excellent | Excellent | Excellent | Excellent | Good |
| | Test for Application of the Patch on a Rat | Amount of Transdermal Absorption ($\mu g/cm^2/24h$) | 135 | 284 | — | — | — | — | — | — | — | — |
| | | Skin Stimulation | Excellent | Excellent | — | — | — | — | — | — | — | — |
| | | Peeling State | Excellent | Good | — | — | Excellent | Excellent | Good | Excellent | Good | Good |
| | | Adhesive Transfer | Good | Excellent | — | — | Good | Excellent | Good | Excellent | Excellent | Excellent |

Table 5

| | | | Comparative Example 1 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 |
|---|---|---|---|---|---|---|---|---|---|---|---|---|
| CONSTRUCTION | DMAE (% by weight) | | 10 | 10 | 10 | 15 | 10 | 10 | 10 | 10 | 10 | 10 |
| | Acrylic Adhesive (% by weight) | A | 90 | — | — | — | 80 | 80 | 80 | 80 | — | — |
| | | B | — | 90 | — | — | — | — | — | — | — | — |
| | | C | — | — | 90 | 65 | — | — | — | — | — | — |
| | | D | — | — | — | — | — | — | — | — | — | — |
| | | E | — | — | — | — | — | — | — | — | 65 | — |
| | | F | — | — | — | — | — | — | — | — | — | — |
| | | G | — | — | — | — | — | — | — | — | — | 65 |
| | | H | — | — | — | — | — | — | — | — | — | — |
| | | I | — | — | — | — | — | — | — | — | — | — |
| | Fatty Acid Ester (% by weight) | Isopropyl Myristate (C13-C3) | — | — | — | 15 | — | — | — | — | 20 | 20 |
| | | Isopropyl Palmitate (C15-C3) | — | — | — | — | — | — | — | — | — | — |
| | Additive (% by weight) | Butylene Glycol | — | — | — | — | 10 | — | — | — | — | — |
| | | Propylene Glycol | — | — | — | — | — | 10 | — | — | — | — |
| | | Octanediol | — | — | — | — | — | — | — | — | — | — |
| | | Monoethanolamine | — | — | — | — | — | — | 10 | — | — | — |
| | | Liquid Paraffin | — | — | — | — | — | — | — | 10 | — | — |
| | | Low Substituted Hydroxypropyl Cellulose | — | — | — | 5 | — | — | — | — | 5 | 5 |
| | | Crosslinked Polyvinyl Pyrrolidone | — | — | — | — | — | — | — | — | — | — |
| | Thickness of Plaster Layer (μm) | | 50 | 50 | 50 | 100 | 50 | 50 | 50 | 50 | 70 | 70 |
| EVALUATION | Stability Test | Residual Rate of DMAE (% by weight) | 96 | 47 | 47 | 55 | 74 | 43 | 73 | 97 | 98 | 97 |
| | Test for Application of the Patch on a Rat | Amount of Transdermal Absorption (μg/cm²/24h) | 42 | — | — | — | 49 | 57 | — | 41 | 91 | 84 |
| | | Skin Stimulation | Excellent | — | — | Poor | Poor | Poor | Poor | Excellent | Excellent | Excellent |
| | | Peeling State | Good | — | — | Excellent | Good | Good | Excellent | Excellent | Good | Poor |
| | | Adhesive Transfer | Excellent | — | — | Poor | Poor | Poor | Excellent | Excellent | Excellent | Excellent |

Table 6

| | | | Comparative Example 11 | Comparative Example 12 | Comparative Example 13 | Comparative Example 14 | Comparative Example 15 | Comparative Example 16 | Comparative Example 17 | Comparative Example 18 | Comparative Example 19 | Comparative Example 20 |
|---|---|---|---|---|---|---|---|---|---|---|---|---|
| CONSTRUCTION | DMAE (% by weight) | | 10 | 10 | 5 | 5 | 5 | 5 | 5 | 5 | 5 | 5 |
| | Acrylic Adhesive (% by weight) | A | — | 65 | — | — | — | — | — | — | — | — |
| | | B | — | — | — | — | — | — | — | — | — | — |
| | | C | — | — | — | — | — | — | — | — | — | — |
| | | D | — | — | 95 | — | — | — | — | — | — | — |
| | | E | — | — | — | — | 95 | — | — | 85 | — | — |
| | | F | — | — | — | 95 | — | — | — | — | — | — |
| | | G | — | — | — | — | — | 95 | — | — | 85 | — |
| | | H | 65 | — | — | — | — | — | 95 | — | — | 85 |
| | | I | — | — | — | — | — | — | — | — | — | — |
| | Fatty Acid Ester (% by weight) | Isopropyl Myristate (C13-C3) | 20 | — | — | — | — | — | — | — | — | — |
| | | Isopropyl Palmitate (C15-C3) | — | — | — | — | — | — | — | 10 | 10 | 10 |
| | Additive (% by weight) | Butylene Glycol | — | — | — | — | — | — | — | — | — | — |
| | | Propylene Glycol | — | — | — | — | — | — | — | — | — | — |
| | | Octanediol | — | 20 | — | — | — | — | — | — | — | — |
| | | Monoethanolamine | — | — | — | — | — | — | — | — | — | — |
| | | Liquid Paraffin | — | — | — | — | — | — | — | — | — | — |
| | | Low Substituted Hydroxypropyl Cellulose | 5 | 5 | — | — | — | — | — | — | — | — |
| | | Crosslinked Polyvinyl Pyrrolidone | — | — | — | — | — | — | — | — | — | — |
| | Thickness of Plaster Layer (μm) | | 70 | 70 | 70 | 70 | 70 | 70 | 70 | 70 | 70 | 70 |
| EVALUATION | Stability Test | Residual Rate of DMAE (% by weight) | 83 | 82 | — | — | — | — | — | — | — | — |
| | Test for Application of the Patch on a Rat | Amount of Transdermal Absorption (μg/cm²/24h) | 28 | 131 | — | — | — | — | — | — | — | — |
| | | Skin Stimulation | Excellent | Poor | — | — | — | — | — | — | — | — |
| | | Peeling State | Excellent | Excellent | — | — | — | — | — | — | — | — |
| | | Adhesive Transfer | Good | Good | — | — | — | — | — | — | — | — |

Industrial Applicability

In the transdermal patch of the present invention, transdermal permeability of DMAEs is improved by a specifically structured fatty acid ester and the fatty acid ester itself acts as a carrier for delivering the DMAEs inside the human skin, so that the transdermal patch exhibits excellent transdermal absorbency of the DMAEs. Therefore, the transdermal patch is advantageously used like as a therapeutic drug for treatment of essential hypotension and orthostatic hypotension.

## Claims

1. A transdermal patch having a support and a plaster layer integrally laminated on one surface of the support, the plaster layer comprising:

   2-amino-1-(2',5'-dimethoxyphenyl) ethanol (also known as desglymidodrine) or pharmacologically acceptable salt thereof;
   40 to 96% by weight of acrylic adhesive prepared by copolymerizing monomers respectively containing 30 to 99% by weight of alkyl methacrylate having an alkyl group with a carbon number of 6 to 22 and 1 to 70% by weight of alkyl acrylate having an alkyl group with a carbon number of 2 to 20; and
   3 to 40% by weight of fatty acid ester prepared by dehydro-condensing saturated fatty acid having an alkyl group with a carbon number of 10 to 20 and saturated aliphatic monohydric alcohol having an alkyl group with a carbon number of 2 to 20,
   wherein solubility of the 2-amino-1-(2', 5'-dimethoxyphenyl) ethanol or the pharmacologically acceptable salt thereof is 0.05 to 5 g at 25°C with respect to 100g of the fatty acid ester.

2. The transdermal patch according to claim 1, wherein a content of the 2-amino-1-(2', 5'-dimethoxyphenyl) ethanol or the pharmacologically acceptable salt thereof is 0.5 to 40% by weight in the plaster layer.

3. The transdermal patch according to claim 1, wherein the acrylic adhesive is prepared by copolymerizing monomers respectively containing 50 to 99% by weight of alkyl methacrylate with an alkyl group having a carbon number of 6 to 22 and 1 to 50% by weight of alkyl acrylate with an alkyl group having a carbon number of 2 to 20.

4. The transdermal patch according to claim 1, wherein the acrylic adhesive is a copolymer prepared by copolymerizing monomers respectively containing 70 to 95% by weight of 2-ethylhexyl methacrylate and 1 to 30% by weight of 2-ethylhexyl acrylate.

5. The transdermal patch according to claim 1, wherein the acrylic adhesive is a copolymer prepared by copolymerizing monomers respectively containing 70 to 95% by weight of 2-ethylhexyl methacrylate, 1 to 20% by weight of 2-ethylhexyl acrylate, and 4 to 29% by weight of dodecyl methacrylate.

6. The transdermal patch according to claim 1, wherein solubility of the 2-amino-1-(2', 5'-dimethoxyphenyl) ethanol or the pharmacologically acceptable salt thereof is 0.1 to 3 g at 25°C with respect to the fatty acid ester.

7. The transdermal patch according to claim 1, wherein a solubility of the 2-amino-1-(2', 5'-dimethoxyphenyl) ethanol or the pharmacologically acceptable salt thereof is 0.1 to 1 g at 25°C with respect to the fatty acid ester.

8. The transdermal patch according to claim 1, wherein the fatty acid ester is at least one kind of fatty acid ester selected from the group consisting of isopropyl myristate, isopropyl palmitate and hexyl laurate.

9. The transdermal patch according to claim 1, wherein a filler is contained in the plaster layer.

10. The transdermal patch according to claim 9, wherein the filler is at least one kind of fillers selected from the group consisting of anhydrous silicic acid, low substituted hydroxypropylcellulose and crosslinked polyvinyl pyrrolidone.

## Patentansprüche

1. Transdermales Pflaster mit einem Träger und einer integral auf einer Oberfläche des Trägers laminierten Pflasterschicht, wobei die Pflasterschicht umfasst:

2-Amino-1-(2',5'-dimethoxyphenyl)ethanol (ebenso als Desglymidodrin bekannt) oder ein pharmakologisch annehmbares Salz davon;
40 bis 96 Gew.-% Acrylklebstoff, hergestellt durch Copolymerisieren von Monomeren, respektive enthaltend 30 bis 99 Gew.-% Alkylmethacrylat mit einer Alkylgruppe mit einer Kohlenstoffanzahl von 6 bis 22 und 1 bis 70 Gew.-% Alkylacrylat mit einer Alkylgruppe mit einer Kohlenstoffanzahl von 2 bis 20; und
3 bis 40 Gew.-% Fettsäureester, hergestellt durch Dehydro-Kondensation gesättigter Fettsäure mit einer Alkylgruppe mit einer Kohlenstoffanzahl von 10 bis 20 und gesättigtem, aliphatischem, einwertigem Alkohol mit einer Alkylgruppe mit einer Kohlenstoffanzahl von 2 bis 20,
wobei die Löslichkeit des 2-Amino-1-(2',5'-dimethoxyphenyl)ethanols oder des pharmakologisch annehmbaren Salzes davon 0,05 bis 5 g bei 25°C beträgt, mit Bezug auf 100 g des Fettsäureesters.

2. Transdermales Pflaster nach Anspruch 1, wobei ein Gehalt des 2-Amino-1-(2',5'-dimethoxyphenyl)ethanols oder des pharmakologisch annehmbaren Salzes davon 0,5 bis 40 Gew.-% in der Pflasterschicht beträgt.

3. Transdermales Pflaster nach Anspruch 1, wobei der Acrylklebstoff hergestellt wird durch Copolymerisieren von Monomeren, respektive enthaltend 50 bis 99 Gew.-% Alkylmethacrylat mit einer Alkylgruppe mit einer Kohlenstoffanzahl von 6 bis 22 und 1 bis 50 Gew.-% Alkylacrylat mit einer Alkylgruppe mit einer Kohlenstoffanzahl von 2 bis 20.

4. Transdermales Pflaster nach Anspruch 1, wobei der Acrylklebstoff ein Copolymer ist, hergestellt durch Copolymerisieren von Monomeren, respektive enthaltend 70 bis 95 Gew.-% 2-Ethylhexylmethacrylat und 1 bis 30 Gew.-% 2-Ethylhexylacrylat.

5. Transdermales Pflaster nach Anspruch 1, wobei der Acrylklebstoff ein Copolymer ist, hergestellt durch Copolymerisieren von Monomeren, respektive enthaltend 70 bis 95 Gew.-% 2-Ethylhexylmethacrylat, 1 bis 20 Gew.-% 2-Ethylhexylacrylat und 4 bis 29 Gew.-% Dodecylmethacrylat.

6. Transdermales Pflaster nach Anspruch 1, wobei die Löslichkeit des 2-Amino-1-(2',5'-dimethoxyphenyl)ethanols oder des pharmakologisch annehmbaren Salzes davon 0,1 bis 3 g bei 25°C mit Bezug auf den Fettsäureester beträgt.

7. Transdermales Pflaster nach Anspruch 1, wobei die Löslichkeit des 2-Amino-1-(2',5'-dimethoxyphenyl)ethanols oder des pharmakologisch annehmbaren Salzes davon 0,1 bis 1 g bei 25°C mit Bezug auf den Fettsäureester beträgt.

8. Transdermales Pflaster nach Anspruch 1, wobei der Fettsäureester mindestens eine Art eines Fettsäureesters ist, gewählt aus der Gruppe, bestehend aus Isopropylmyristat, Isopropylpalmitat und Hexyllaurat.

9. Transdermales Pflaster nach Anspruch 1, wobei ein Füllstoff in der Pflasterschicht enthalten ist.

10. Transdermales Pflaster nach Anspruch 9, wobei der Füllstoff mindestens eine Art von Füllstoffen ist, gewählt aus der Gruppe, bestehend aus wasserfreier Kieselsäure, niedrigsubstituierter Hydroxypropylcellulose und vernetztem Polyvinylpyrrolidon.

## Revendications

1. Timbre transdermique ayant un support et une couche de pansement stratifiée en une seule pièce sur une surface du support, la couche de pansement comprenant :

du 2-amino-1-(2',5'-diméthoxyphényl)éthanol (également connu sous le nom de desglymidodrine) ou un sel pharmacologiquement acceptable de celui-ci ;
de 40 à 96 % en poids d'un adhésif acrylique préparé par copolymérisation de monomères contenant respectivement de 30 à 99 % en poids de méthacrylate d'alkyle ayant un groupe alkyle avec un nombre d'atomes de carbone de 6 à 22 et de 1 à 70 % en poids d'acrylate d'alkyle ayant un groupe alkyle avec un nombre d'atomes de carbone de 2 à 20 ; et
de 3 à 40 % en poids d'ester d'acide gras préparé par déshydro-condensation d'acide gras saturé ayant un groupe alkyle avec un nombre d'atomes de carbone de 10 à 20 et d'alcool monohydrique aliphatique saturé ayant un groupe alkyle avec un nombre d'atomes de carbone de 2 à 20,

dans lequel la solubilité du 2-amino-1-(2',5'-diméthoxyphényl)éthanol ou de son sel pharmacologiquement acceptable est de 0,05 à 5 g à 25°C relativement à 100 g de l'ester d'acide gras.

2. Timbre transdermique selon la revendication 1, dans lequel une teneur en 2-amino-1-(2',5'-diméthoxyphényl)éthanol ou son sel pharmacologiquement acceptable est de 0, 5 à 40 % en poids dans la couche de pansement.

3. Timbre transdermique selon la revendication 1, dans lequel l'adhésif acrylique est préparé par copolymérisation de monomères contenant respectivement de 50 à 99 % en poids de méthacrylate d'alkyle avec un groupe alkyle ayant un nombre d'atomes de carbone de 6 à 22 et de 1 à 50 % en poids d'acrylate d'alkyle avec un groupe alkyle ayant un nombre d'atomes de carbone de 2 à 20.

4. Timbre transdermique selon la revendication 1, dans lequel l'adhésif acrylique est un copolymère préparé par copolymérisation de monomères contenant respectivement de 70 à 95 % en poids de méthacrylate de 2-éthylhexyle et de 1 à 30 % en poids d'acrylate de 2-éthylhexyle.

5. Timbre transdermique selon la revendication 1, dans lequel l'adhésif acrylique est un copolymère préparé par copolymérisation de monomères contenant respectivement de 70 à 95 % en poids de méthacrylate de 2-éthylhexyle, de 1 à 20 % en poids d'acrylate de 2-éthylhexyle, et de 4 à 29 % en poids de méthacrylate de dodécyle.

6. Timbre transdermique selon la revendication 1, dans lequel la solubilité du 2-amino-1-(2',5'-diméthoxyphényl)éthanol ou de son sel pharmacologiquement acceptable est de 0,1 à 3 g à 25°C relativement à l'ester d'acide gras.

7. Timbre transdermique selon la revendication 1, dans lequel une solubilité du 2-amino-1-(2',5'-diméthoxyphényl) éthanol ou de son sel pharmacologiquement acceptable est de 0,1 à 1 g à 25°C relativement à l'ester d'acide gras.

8. Timbre transdermique selon la revendication 1, dans lequel l'ester d'acide gras est au moins une sorte d'ester d'acide gras choisi dans le groupe constitué par le myristate d'isopropyle, le palmitate d'isopropyle et le laurate d'hexyle.

9. Timbre transdermique selon la revendication 1, dans lequel une charge est contenue dans la couche de pansement.

10. Timbre transdermique selon la revendication 9, dans lequel la charge est au moins une sorte de charges choisies dans le groupe constitué par l'acide silicique anhydre, l'hydroxypropylcellulose faiblement substituée et la polyvinylpyrrolidone réticulée.

FIG. 1

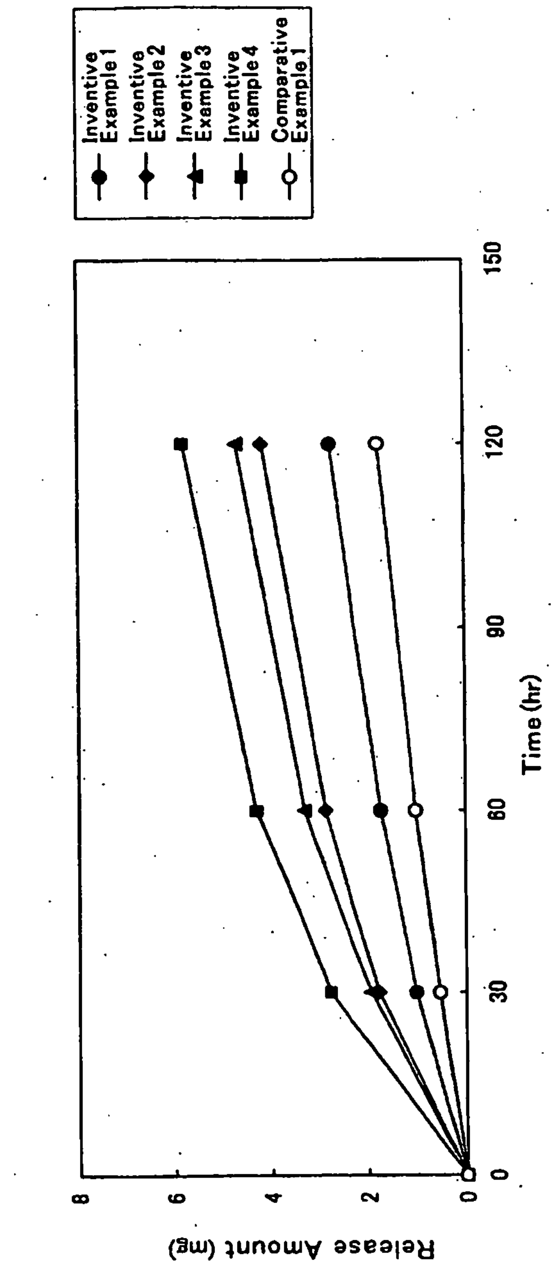
Inventive Example1
Inventive Example2
Inventive Example3
Inventive Example4
Comparative Example1
Release Amount (mg)
Time (hr)
0
2
4
6
8
0
30
60
90
120
150

FIG. 2

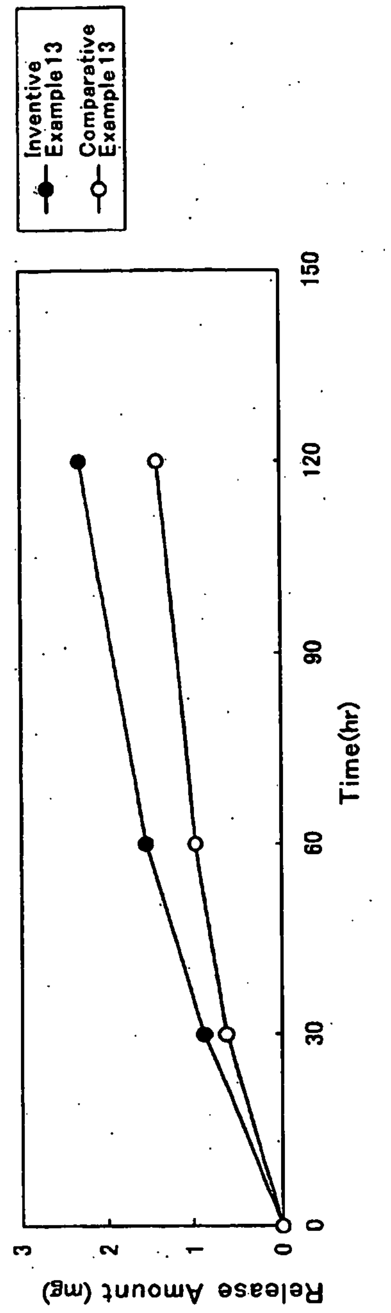
Inventive Example 13
Comparative Example 13
Release Amount (mg)
0
1
2
3
0
30
60
90
120
150
Time(hr)

FIG. 3

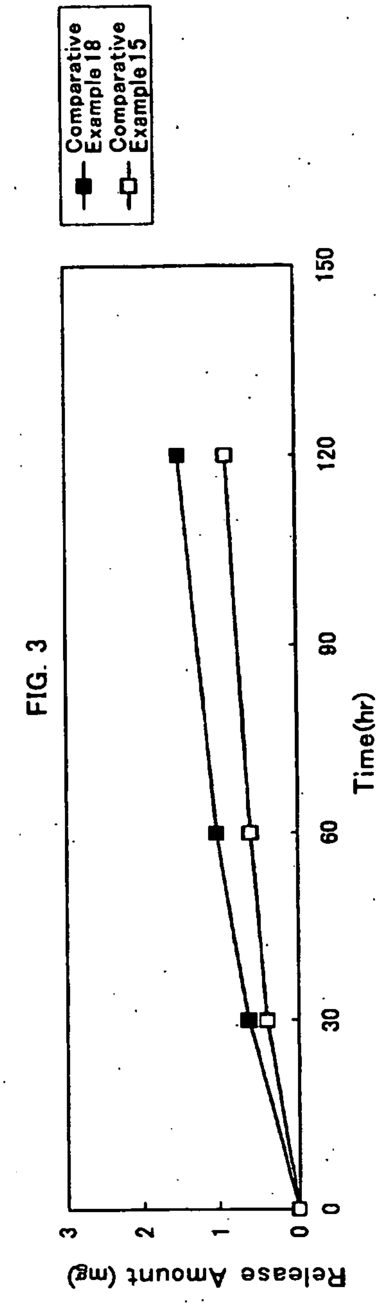
FIG. 3
Comparative Example18
Comparative Example15
Release Amount (mg)
0
1
2
3
0
30
60
90
120
150
Time(hr)

FIG. 4

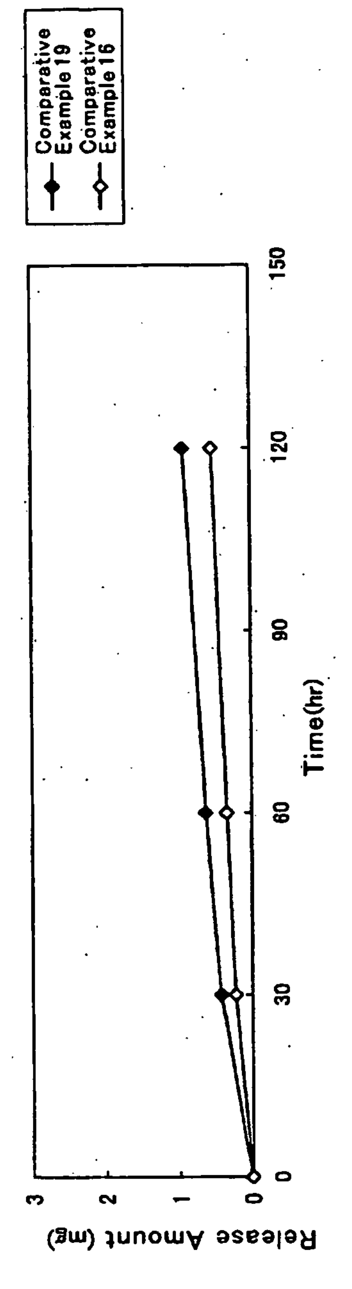
Comparative Example 19
Comparative Example 16
Release Amount (mg)
3
2
1
0
0
30
60
90
120
150
Time(hr)

FIG. 5

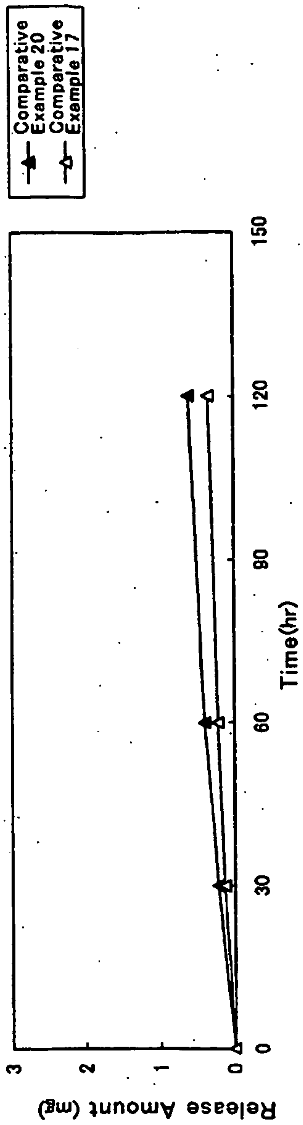
Comparative Example 20
Comparative Example 17
Release Amount (mg)
3
2
1
0
0
30
60
90
120
150
Time(hr)

FIG. 6

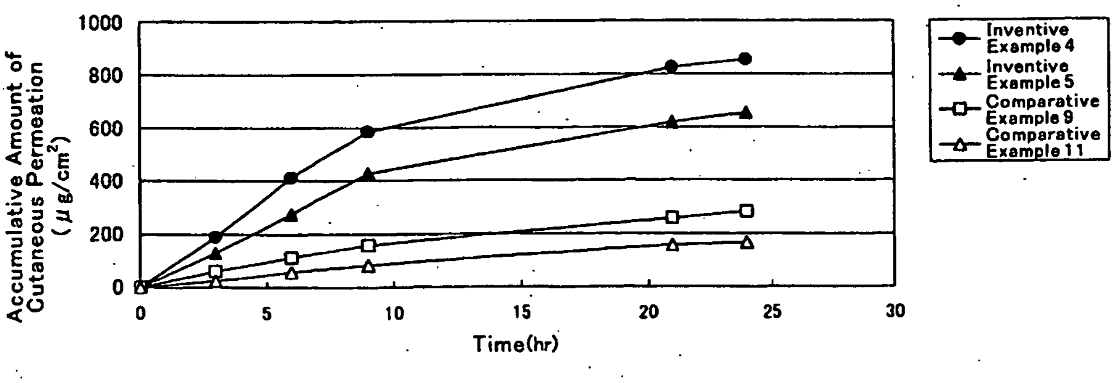
Accumulative Amount of Cutaneous Permeation
( μg/cm2)
1000
800
600
400
200
0
0
5
10
15
20
25
30
Time(hr)
Inventive Example 4
Inventive Example 5
Comparative Example 9
Comparative Example 11

FIG. 7

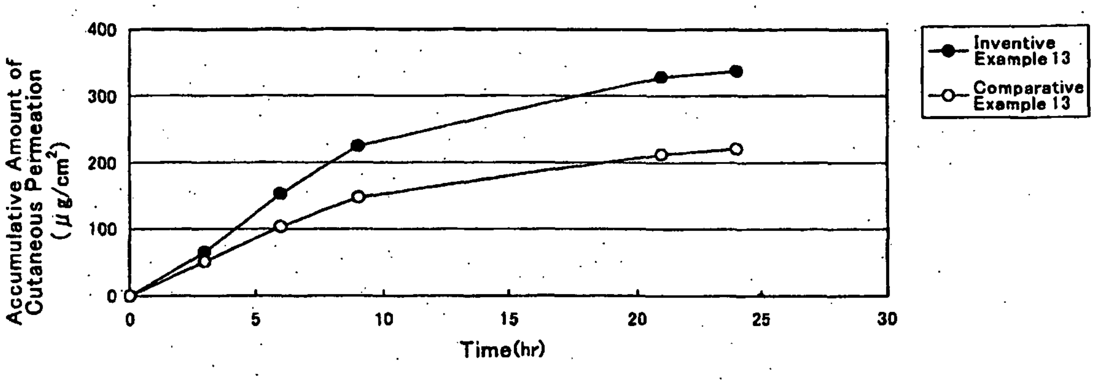


FIG. 8

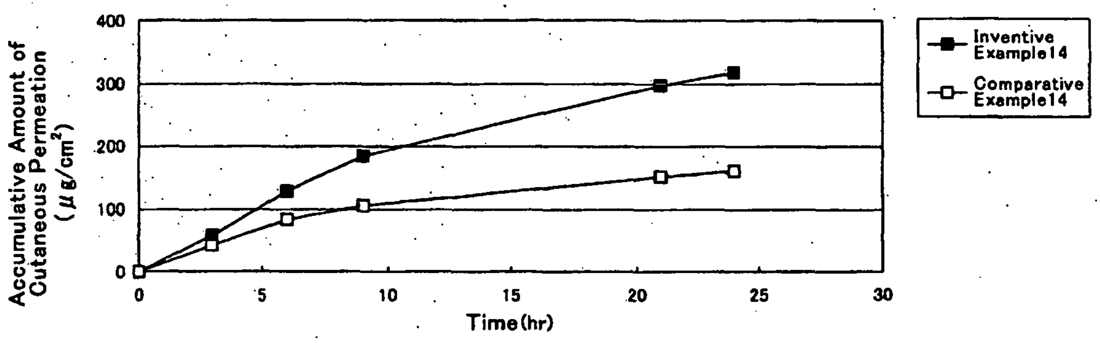


FIG. 9

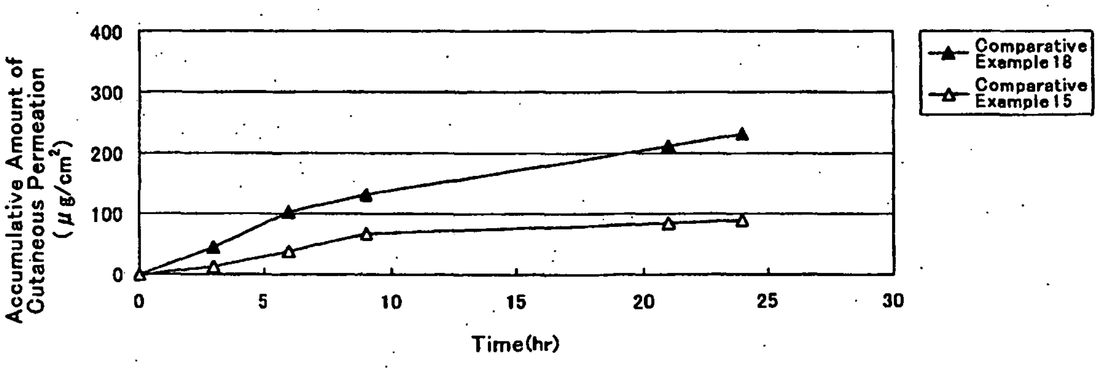


FIG. 10

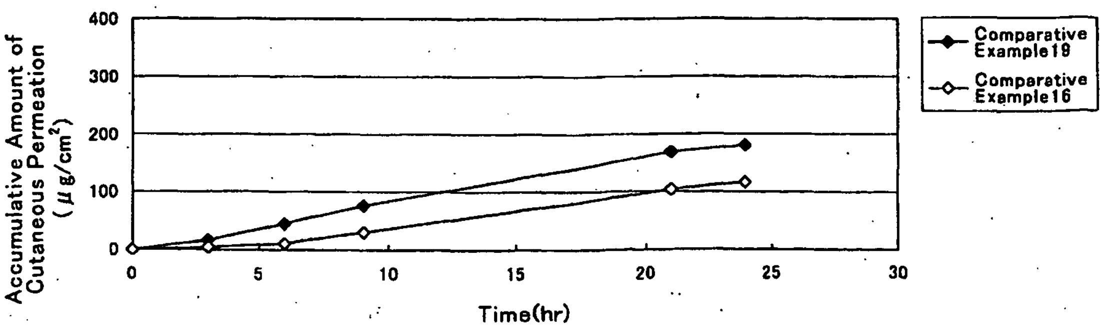

FIG. 11

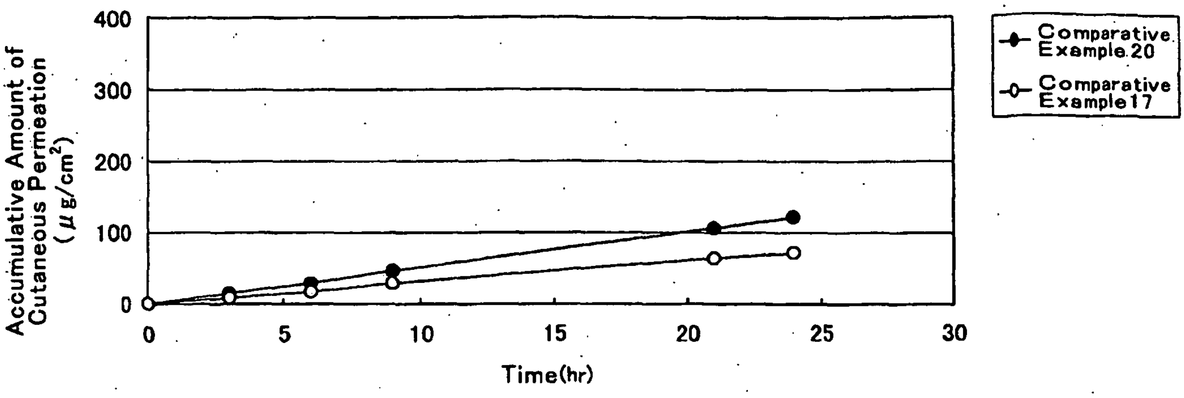
Accumulative Amount of Cutaneous Permeation (μg/cm2)
400
300
200
100
0
0
5
10
15
20
25
30
Time(hr)
Comparative Example 20
Comparative Example 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3571511 B
- JP 2003300873 A
- EP 1498118 A